Europäisches Patentamt

European Patent Office

Office européen des brevets

(11) Publication number: **0 046 290**
**B1**

(12) **EUROPEAN PATENT SPECIFICATION**

(45) Date of publication of patent specification: **02.01.85**

(21) Application number: **81106371.8**

(22) Date of filing: **17.08.81**

(51) Int. Cl.⁴: **A 61 K 31/415** //
**(A61K31/415, 31/415)**

(54) **Synergistic combination of (R,S)alpha-fluoromethylhistidine and cimetidine as an anti-ulcer agent.**

(30) Priority: **18.08.80 US 179358**

(43) Date of publication of application:
**24.02.82 Bulletin 82/08**

(45) Publication of the grant of the patent:
**02.01.85 Bulletin 85/01**

(84) Designated Contracting States:
**CH DE FR GB IT LI NL**

(56) References cited:
**US-A-4 000 302**
**US-A-4 215 221**

(73) Proprietor: **MERCK & CO. INC.**
**126, East Lincoln Avenue P.O. Box 2000**
**Rahway New Jersey 07065 (US)**

(72) Inventor: **Kollonitsch, Janos**
**3 Plymouth Road**
**Westfield New Jersey 07090 (US)**

(74) Representative: **Abitz, Walter, Dr.-Ing. et al**
**Abitz, Morf, Gritschneder P.O. Box 86 01 09**
**D-8000 München 86 (DE)**

Courier Press, Leamington Spa, England.

EP 0 046 290 B1

**0 046 290**

## Description

### Background of the invention

(R,S)$\alpha$-Fluoromethylhistidine is a known compound that is known to inhibit mammalian histidine decarboxylase *in vivo*. In consequence, histamine levels decrease considerably and for extended periods. Cimetidine N-cyano-N'-methyl-N''-[2-[[(5-methyl-1H-imidazol-4-yl)-methyl]-thio]ethyl]-quanidine, is a known H2 receptor inhibitor that is used in the treatment of gastric ulcers. Cimetidine acts at the histamine receptor sites but does not appreciably influence histamine levels. The instant combination of (R,S)$\alpha$-fluoromethylhistidine and cimetidine has been found to synergistically reduce the levels of histamine and incidence of gastric ulcers at lower doses and for longer durations than is possible for either drug alone.

### Summary of the invention

The instant invention is concerned with the synergistic combination of (R,S)$\alpha$-fluoromethyl-histidine and cimetidine for use in the treatment of excess gastric acid secretion and gastric ulcers. Thus, it is an object of this invention to describe such a combination. It is a further object of this invention to describe the synergistic effects observed when such a combination is administered to animals. A further object is to describe the pharmaceutical composition containing the synergistic combination.

### Description of the invention

(R,S)$\alpha$-Fluoromethylhistidine is known to reduce the levels of histamine when administered. Cimetidine is known to reduce gastric acid secretion and the incidence of gastric ulcers. It has been surprisingly discovered that the administration of a combination of (R,S)$\alpha$-fluoromethylhistidine and cimetidine has the effects, not only of simultaneously reducing histamine levels and gastric acid secretions, but also of reducing such levels beyond the potential of either drug alone, and even more surprising, of reducing side effects such as gastric acid rebound, and permitting lower doses of both drugs to achieve the same effects, with the added advantage of less toxicity.

In a test of the ability to reduce gastric lesions, (R,S)$\alpha$-fluoromethylhistidine and cimetidine were tested alone and in combination in rats in the cold restraint test of induced lesions. In this test rats are restrained for 3 hours in a rigid cylinder of 40 mm inside diameter at 8°C. Under these conditions, a 100% incidence of gastric lesions is observed. The ability of a drug to inhibit the induction of gastric lesions can thus be readily measured.

In a test of (R,S)$\alpha$-fluoromethylhistidine 100 mg per kg intraperitonealy resulted in lesions present in 4 out of 6 animals with a total number of lesions of 24. Control animals had lesions in 6 out of 6 animals and a total lesion count of 70. Cimetidine at 100 mg per kg in the same test had lesions present in 3 out of 6 animals and a total of 29 lesions. The control animals had lesions in 6 out of 6 animals and a total count of 29 lesions.

In combinations at 30, 60 and 100 mg of each drug the incidence of lesions was 4 out of 6, 2 out of 6 and 2 out of 7 animals respectively, with a total count of lesions of 13, 20 and 13 respectively. The control animals (two groups) had an incidence of 6 out of 6 or 7 out of 7 animals and a total lesion count of 35 and 54 respectively.

In the area of gastric lesions, both (R,S)$\alpha$-fluoromethylhistidine and cimetidine individually reduce the incidence and total number of gastric lesions. However the combination, even at combined doses lower than the individual doses, has a greater effect than either drug alone. This is particularly observed at 30 mg per kg of each drug reducing the total number of lesions to 13 while the individual compounds at 100 mg per kg could reduce the total lesions to 24 and 29 for (R,S)$\alpha$-fluoromethyl-histidine and cimetidine respectively.

Thus, it is apparent that the combination of (R,S)$\alpha$-fluoromethylhistidine and cimetidine is a potent synergistic combination for the reduction of histamine levels and the treatment of excess gastric acid secretions and gastric ulcers which properties are not expected from the properties of the individual compounds.

It will be appreciated also that $\alpha$-fluoromethylhistidine may be combined with other histamine H2 receptor inhibitors such as ranitidine, burimimide, metiamide, and the like and histamine H1 receptors such as diphenhydramide, dimenhydrinate, tripeleneamine hydrochloride, pyrilamine, methapyriline, chlorpheniramine, cyclizine hydrochloride, cyproheptadine, and the like. Such combinations will be useful for the treatment of allergic reactions, gastric ulcers, inflammation, and other conditions treated with H1 and H2 receptor antagonists.

The combination of $\alpha$-fluoromethylhistidine and cimetidine may be administered orally, topically or parenterally or by suppository. The preferred route is oral administration. They will in general be associated with a pharmaceutically acceptable carrier or diluent to provide a suitable pharmaceutical composition.

For oral administration the pharmaceutical composition can most conveniently be in the form of capsules or tablets which may be slow release tablets. The compositions may also take the form of a dragee or may be in syrup form. Suitable topical preparations include ointments, lotions, creams, powders and sprays.

2

A convenient daily dose by the oral route would be of the order of from 20 to 2000 mg per kg per day of (R,S)α-fluoromethylhistidine in combination with from 20 to 2000 mg per kg per day of cimetidine. Preferred levels would be from about 50 to 500 mg per kg per day of each drug. The compounds are conveniently administered in unit dosage forms in single or multiple divided doses. The dosage units would contain from 5 to 200 mg of α-fluoromethylhistidine and from 5 to 200 mg of cimetidine and a convenient regime would call for the administration of one or two of such dosage units from one to four times a day.

Within the above ranges, it is preferred that the ratio of (R,S)α-fluoromethylhistidine to cimetidine range from 1:5 to 5:1, and even more preferably the synergistic combination of the two drugs would be about 1:1.

For topical application a spray, ointment, cream or lotion may be used containing from 1/2 to 2% of each of (R,S)α-fluoromethylhistidine and cimetidine.

Typical pharmaceutical dosage forms useful for the administration of the instant combination of α-fluoromethylhistidine and cimetidine are given in the following examples.

It has been found that the racemic mixture of (R) and (S)-α-fluoromethylhistidine may be separated into the separate (R) and (S) optical isomers. In addition, the (S) isomer has been found to be much more active than the (R) isomer. The isomers are separated using variations of known techniques used for the separation of optical isomers. Generally techniques such as the preparation of optically active derivatives and salts of the (R,S) racemic mixture, and stereo selective isolation procedures such as fractional crystallization are employed.

### Example 1

| Capsule ingredients | Amounts |
|---|---|
| (R,S)α-Fluoromethylhistidine | 100 mg |
| Cimetidine | 100 mg |
| Lactose | 100 mg |

The ingredients are mixed and filled into a hard gelatin capsules.

| | |
|---|---|
| (R,S)α-Fluoromethylhistidine | 100 mg |
| Cimetidine | 100 mg |
| Sucrose | 75 mg |
| Starch | 25 mg |
| Talc | 5 mg |
| Stearic Acid | 2 mg |

The ingredients are mixed and filled into hard gelatin capsules.

### Example 2
For 100 tablets containing 100 mg of (R,S)α-fluoromethylhistidine and 100 mg of cimetidine.

| Tablet ingredients | Amounts |
|---|---|
| (R,S)α-Fluoromethylhistidine | 10 g |
| Cimetidine | 10 g |
| Anhydrous Lactose USP | 21.7 g |
| Starch (directly compressable) | 3 g |
| Magnesium Stearate | 0.3 g |

The drugs are sieved through a 250 $\mu$m sieve and all of the solids are intimately mixed in a blender and compressed between 8.5 mm diameter punches in a tableting machine.

# 0 046 290

## Example 3

| Oral syrup ingredients | Amounts |
|---|---|
| (R,S)$\alpha$-Fluoromethylhistidine | 1.0% w/v |
| Cimetidine | 1.0% w/v |
| Sorbitol Solution | 60.0% v/v |
| Dilute Hydrochloric Acid | as required |
| Flavoring | as required |
| Distilled Water | q.s. to 100% |

The drugs are dissolved in water and the pH adjusted as necessary with the dilute hydrochloric acid. The sorbital solution, flavoring and the rest of the water added and the pH adjusted if necessary. The syrup is clarified by filtration through suitable cellulosic filter pads.

Example 4
A. Preparation of ($\pm$)-methyl $\alpha$-fluoromethylhistidinate 1,5-naphthalene disulfonate

Into a Fisher-Porter tube is charged 5 g of ($\pm$) $\alpha$-fluoromethylhistidine hydrochloride, 100 ml of methanol, 45 ml of methyl orthoformate and 9.7 g of naphthalene-1,5-disulfonic acid. The tube is sealed and heated in a steam bath overnight. The tube is cooled in an ice bath and the crystalline product (naphthalene-1,5 disulfonic acid salt of $\alpha$-fluoromethylhistidine) is filtered and returned to the Fisher-Porter tube. Methanol (120 ml), methyl orthoformate (55 ml), and naphthalene-1,5 disulfonic acid (9.4 g) are added, the tube sealed and heated overnight in a steam bath, then cooled in an ice bath for *ca.* 5 hours. The crystals of ($\pm$)-methyl $\alpha$-fluoromethylhistidinate 1,5-naphthalene disulfonate are filtered, washed with cold methanol and diethyl ether and dried *in vacuo*.

B. Preparation of ($\pm$)-methyl $N_{im}$ trityl $\alpha$-fluoromethylhistidinate

($\pm$)-Methyl $\alpha$-fluoromethylhistidinate-1,5-naphthalene disulfonate (490 mg) is added to 5 ml of methylene dichloride, trityl chloride (586 mg) and triethylamine (0.6 ml) is added. After stirring overnight at room temperature, the reaction mixture is filtered, the filtrate is extracted with water, the organic phase is dried over $MgSO_4$ and evaporated *in vacuo*. The residue represented the N,N-ditrityl derivative of ($\pm$)-methyl $\alpha$-fluoromethylhistidinate. For selective detritylation first an HCl solution of diethyl ether is prepared by introducing 1.2 g of HCl gas into 13 g of diethyl ether. The ditrityl compound is suspended in 15 ml of diethyl ether and the above HCl-diethyl ether solution added into it. The mixture is heated to reflux with stirring for 1 hour, cooled, filtered and washed with diethyl ether, to give 480 mg of the hydrochloride of ($\pm$) methyl-$N_{im}$-trityl-$\alpha$-fluoromethylhistidinate. 300 Mg of this product is suspended in $CH_2Cl_2$, saturated aqueous $NaHCO_3$ solution added and the mixture is stirred for a few minutes. The separated $CH_2Cl_2$ layer is treated again with $NaHCO_3$, dried over $MgSO_4$, and evaporated *in vacuo* to give an oil which crystallized from tetrahydrofuran, to give the tetra-hydrofuranate of ($\pm$) methyl $N_{im}$ trityl $\alpha$-fluoromethylhistidinate, mp 129—131°. According to C—H—N—F analysis and PMR spectrum, this compound is a tetrahydrofuran complex of methyl $N_{im}$ trityl $\alpha$-fluoromethylhistidinate, containing 0.8 mol of tetrahydrofuran.

C. Preparation of d-$\alpha$-Bromocamphor-$\pi$-sulfonic acid from $NH_4$ salt

72.3 G of *d*-$\alpha$-bromocamphor-$\pi$-sulfonic acid $NH_4$ salt (Aldrich, Catalog No. B-6,000-2) is dissolved in 450 ml of water, charcoaled with 3 g of DARCO-G-60, and charged onto a cation-exchange resin column (300 ml of AG-50-X4, 100—200 mesh) in the H+ form. The effluent is collected and evaporated *in vacuo* to give a solid product, with the approximate composition of a sesquihydrate of *d*-$\alpha$-bromocamphor-$\pi$-sulfonic acid.

D. Preparation of (S) (—) Methyl-$N_{im}$-trityl $\alpha$-fluoromethylhistidinate

($\pm$)-Methyl $N_{im}$-trityl $\alpha$-fluoromethylhistidinate tetrahydrofuran complex (27 g) and *d*-$\alpha$-bromocamphor-$\pi$-sulfonic acid sesquihydrate (18.3 g) are added to 650 ml of ethyl acetate with stirring. After 10 minutes of stirring, 0.7 g of seed crystals of the salts of (S) (—)-methyl $N_{im}$ trityl $\alpha$-fluoromethylhistidinate with *d*-$\alpha$-bromocamphor-$\pi$-sulfonic acid are added, followed by 13 ml of water. The stirring is stopped after about one minute. After 2 hours standing at room temperature, the crystals formed are filtered and washed with ethyl acetate (2×50 ml). After drying *in vacuo*, 20.8 g (99% of theory) of (S) (—)-methyl $N_{im}$ trityl $\alpha$-fluoromethylhistidinate . *d*-$\alpha$-bromocamphor-$\pi$-sulfonic acid . $H_2O$ salt are obtained. This salt is purified by recrystallization from 1800 ml of ethyl acetate, seeded with 0.5 g of optically pure salt. After standing overnight, the crystals separated are collected,

4

washed with ethyl acetate (2×200 ml) and dried *in vacuo* at 60°C; 14.6 g of optically pure salt (monohydrated) are obtained, overall yield 69% of theory.

E. Preparation of (S) (−) methyl $N_{im}$-trityl $\alpha$-fluoromethylhistidinate

Seventy and 8/10 g of the optically pure salt, obtained as described above, are suspended in 300 ml of dichloromethane and with stirring, 200 ml of 4% aqueous NaHCO₃ solution are added. After separation of the 2 layers, the lower layer is extracted 3 more times with NaHCO₃ solution; then dried over MgSO₄ and evaporated *in vacuo* to dryness. 200 Ml of diethyl ether are added to the residue to cause crystallization. The crystals are filtered, washed with ether and dried *in vacuo*. 98% yield (theory) of (S) (−) methyl $N_{im}$-trityl $\alpha$-fluoromethylhistidinate is obtained. $[\alpha]_D$ in CHCl₃:— 15.3°.

The seed crystals mentioned in step *D.* of this example were obtained as follows: To a solution of 19.8 g of (±) methyl $N_{im}$ trityl $\alpha$-fluoromethylhistidinate tetrahydrofuranate in 350 ml of ethyl acetate, 12 g of *d-α*-bromocamphor-$\pi$-sulfonic acid is added. After stirring one hour, the small amount of precipitate is filtered off and 3 ml water are added to the mother liquor. After 4 hours stirring, the crystals formed are filtered and recrystallized from a mixture of ethyl acetate (600 ml) and water (6 ml). After standing overnight at room temperature, needles are formed. This salt (needles) is filtered and recrystallized 2 more times from ethyl acetate-water (100:1, vol/vol.

F. Preparation of (S)-(+)-$\alpha$-fluoromethylhistidine dihydrochloride

Thirty-eight and 2/10 g of (S) (−) methyl-$N_{im}$-trityl $\alpha$-fluoromethylhistidinate is added to 600 ml of conc. aq. HCl; this mixture is stirred and heated under a reflux condenser at 95°C (oil bath). (Caution: Strong foaming at the beginning). After 6 hours heating, the mixture is aged overnight at room temperature, filtered, washed with water and the filtrate evaporated *in vacuo* to give (S) (+)-$\alpha$-fluoromethylhistidine dihydrochloride; for purification, this is dissolved in 110 ml of methanol, filtered and while boiling hot, 170 ml of isopropanol added to the filtrate; after aging it in the refrigerator for 3 days, the crystals are filtered and dried *in vacuo*; these crystals represent optically pure (S) (+)-$\alpha$-fluoromethylhistidine dihydrochloride; $[\alpha]_D^{23}$:+17.3° (c, 2.52 in trifluoroacetic acid:water, 1:1); mp 217—219C, with decomposition. Single crystal X-ray diffraction analysis affirms the assigned (S) absolute configuration. The free base (S) (−)-$\alpha$-fluoromethylhistidine [[$\alpha$]$_D^{23}$:−15.7° (c, 2 in CHCl₃)] is obtained from this salt by conventional neutralization.

When Step *D.* is carried out using dry ethyl acetate with no water added, a salt of (S) methyl-$N_{im}$ trityl-$\alpha$-fluoromethylhistidinate with *d-α*-bromocamphor-$\pi$-sulfonic acid is obtained in 6—7% yield. This salt, unlike the salt of part D, which contains equimolar amounts of each component, consist of one mole of (S)-methyl-$N_{im}$-trityl-$\alpha$-fluoromethylhistidinate and 2 moles of d-$\alpha$-bromocamphor-$\pi$-sulfonic acid. In addition, the instant salt is hydrated, while the above prepared salt is not. Treatment of this salt with aqueous NaHCO₃/ethyl ether followed by appropriate extraction, drying and evaporation yields S-methyl-$N_{im}$-trityl-$\alpha$-fluoromethylhistidinate, $[\alpha]_D$ in CDCl₃=—14.3°.

## Claims

1. A composition useful for reducing histamine levels, gastric acid secretions and gastric ulcers in animals which comprises a synergistic combination of (a) (R,S)$\alpha$-fluoromethylhistidine and (b) N-cyano-N′-methyl-N″-[2-[[(5-methyl-1H-imidazol-4-yl)-methyl]-thio]ethyl]guanidine (Cimetidine) and a pharmaceutically acceptable carrier wherein the ratio of component (a) to component (b) is from 5:1 to 1:5.

2. The composition of Claim 1 wherein the ratio of (a) to (b) is about 1:1.

3. The composition of Claim 1 which is intended for oral administration.

4. A synergistic combination for use as a gastric acid secretion inhibitor of (R,S)$\alpha$-fluoromethyl-histidine and N-cyano-N′methyl-N″-[2-[[(5-methyl-1H-imidazol-4-yl)-methyl]thio]ethyl]guanidine wherein the two components are present in the ratio of from 1:5 to 5:1.

5. The synergistic combination of Claim 4 wherein the ratio of the two components is about 1:1.

6. The composition of Claim 4 wherein (S)-$\alpha$-fluoromethylhistidine is employed.

## Revendications

1. Une composition utile pour réduire les taux d'histamine, les sécrétions d'acide gastrique et les ulcères gastriques chez les animaux qui comprend une combinaison synergique de (a) la (R,S)$\alpha$-fluoro-méthylhistidine et (b) la N-cyano-N′-méthyl-N″-[2-[[(5-méthyl-1H-imidazole-4-yl)-méthyl]-thio]éthyl]guanidine (cimétidine) et un support acceptable en pharmacie dans laquelle le rapport du composant (A) au composant (b) est de 5/1 à 1/5.

2. La composition de la revendication 1 dans laquelle le rapport de (a) à (b) est d'environ 1/1.

3. La composition de la revendication 1 qui est destinée à l'administration orale.

4. Une combinaison synergique pour l'emploi comme inhibiteur de la sécrétion d'acide gastrique de (R,S)$\alpha$-fluorométhylhistidine et de N-cyano-N′-méthyl-N″-[2-[[(5-méthyl-1H-imidazole-4-yl)-

méthyl]thio]éthyl]guanidine dans laquelle les deux composants sont présents dans le rapport de 1/5 à 5/1.

5. La composition synergique de la revendication 4 dans laquelle le rapport des deux composants est d'environ 1/1.

6. La composition de la revendication 4 dans laquelle on emploie la (S)α-fluorométhylhistidine.

**Patentansprüche**

1. Eine Zusammensetzung, die zur Verringerung von Histaminspiegeln, Magensäuresekretionen und Magengeschwüren in Tieren brauchbar ist, umfassend eine synergistische Kombination von (a) (R,S)α-Fluormethylhistidin und (b) N-Cyano-N'-methyl-N"-[2-[[(5-methyl-1H-imidazol-4-yl)-methyl]-thio]ethyl]guanidin (Cimetidin) und einem pharmazeutisch annehmbaren Träger, worin das Verhältnis der Komponente (a) zur Komponente (b) 5:1 bis 1:5 beträgt.

2. Die Zusammensetzung des Anspruchs 1, worin das Verhältnis von (a) zu (b) etwa 1:1 ist.

3. Die Zusammensetzung des Anspruchs 1, die für orale Verabreichung bestimmt ist.

4. Eine synergistische Kombination zur Verwendung als Magensäuresekretionsinhibitor aus (R,S)α-Fluormethylhistidin und N-Cyano-N'-methyl-N"-[2-[[(5-methyl-1H-imidazol-4-yl)-methyl]-thio]ethyl]guanidin, worin die zwei Komponenten im Verhältnis 1:5 bis 5:1 vorliegen.

5. Die synergistische Kombination des Anspruchs 4, worin das Verhältnis der zwei Komponenten etwa 1:1 beträgt.

6. Die Zusammensetzung des Anspruchs 4, worin (S)-α-Fluormethylhistidin angewendet wird.